# EUROPEAN PATENT APPLICATION

(11) **EP 1 048 277 A2**
(43) Date of publication of application: **02.11.2000**
(21) Application number: 99120132.8
(22) Date of filing: 08.10.1999
(51) Int. Cl.: A61F 13/472

(54) **Sanitary towel particularly suitable in preventing the menstrual flow to penetrate between the anal area**

(30) Priority: 30.04.1999 IT BA990017
(71) Applicant: Calia, Iolanda, 72100 Brindisi (IT)
(72) Inventor: Calia, Iolanda, 72100 Brindisi (IT)
(74) Representative: Russo, Saverio, Dott. Ing.

(57) **Abstract**

The sanitary towel is formed by an absorbent layer (1) on which is superimposed an absorbent strip (3) attached on one extremity on the intermediate area of said layer (1). The strip has a width d of some centimetres and length L to reach the posterior border (1.3) of layer (1); its inferior surface not sealed to layer (1) permits its lifting.

In a preferred embodiment the strip (3) is an extension of second absorbing layer (2) superimposed and sealed on the anterior surface of the absorbing layer (1).

## Description

This invention is referred to a sanitary towel that prevents the menstrual flow from penetrating between the anal area, and more particularly, that prevents the formation of tedious blood spots on the panties at the anal area, especially if one is lying on her back, and consequently on the sheets during the night in the menstrual period.

As known, one of the biggest problems for women during the menstrual period is to protect, by means of the sanitary towels, their intimate underwear and sheets from undesirable and tedious blood spots.

These anaesthetic spots become a real problem when the woman is lying on her back, as the blood is not drained towards the internal part of the sanitary towel but tends to penetrate between the anal area, in this way both the intimate underwear at its posterior part and the sheets are blood-stained during the night, especially in the case of women having very abundant menstruation (for example after a delivery) or for those having a long period in hospital.

The state of art includes various products that try to solve this problem, among these:
a) special sanitary towels for the night with an absorbent layer thicker than the media, but that present the inconvenient of not being really comfortable and anyway don't specifically solve the problem of the posterior spots;
b) special sanitary towels for the night with "living" wings: the expression refers to sanitary towels that are longer nearly 30% respect to the ordinary ones, and a bit larger at the posterior extremity, provided with lateral elasticated wings permitting a better sticking of the sanitary towel to the panties during the nocturnal movements, said sanitary towels solve the problem of the lateral and posterior spots in case of light flows, but present the inconvenient of not being adapted to very abundant flows and obligate the woman to wear very high panties at their posterior part;
c) sanitary towels provided with two lateral longitudinal little canals for draining the blood inside them, that present the inconvenient of not solving the problem of the posterior spots;
d) tampons that solve both the problems of lateral and posterior spots but present three inconvenients:
   1) necessity of changing them each four hours, so not very comfortable during the night;
   2) impossibility to use them after a surgical operation or soon after a delivery;
   3) strong psychological reticence that many women have in using this kind of internal protection

Main aim of the present invention is to offer to all women a sanitary towel that prevents the menstrual flow to penetrate between the anal area, especially during the night and, anyway, anytime a woman needs to lie on her back, permitting, in a comfortable and effective way, to drain the blood towards the internal part of the sanitary towel, without provoking any posterior blood spots on the panty and/or sheets.

The sanitary towel is described in a detailed way in the following and is illustrated with the figures of the attached sheets of drawings in some preferred embodiments. More particularly,
Fig. 1 illustrates a first embodiment of the sanitary towel according to the invention;
fig. 2 the longitudinal cross-section A-A;
fig. 3 the transversal cross-sections B-B and C-C;
fig. 4, a second embodiment of the sanitary towel according to the invention;
fig. 5, the longitudinal cross-section D-D of fig. 4;
fig. 6, the transversal cross-sections E-E and F-F;
fig. 7, a third embodiment of the sanitary towel according to the invention;
fig. 8, the longitudinal cross-section G-G of fig. 7;
fig. 9, the transversal cross-sections H-H and I-I.

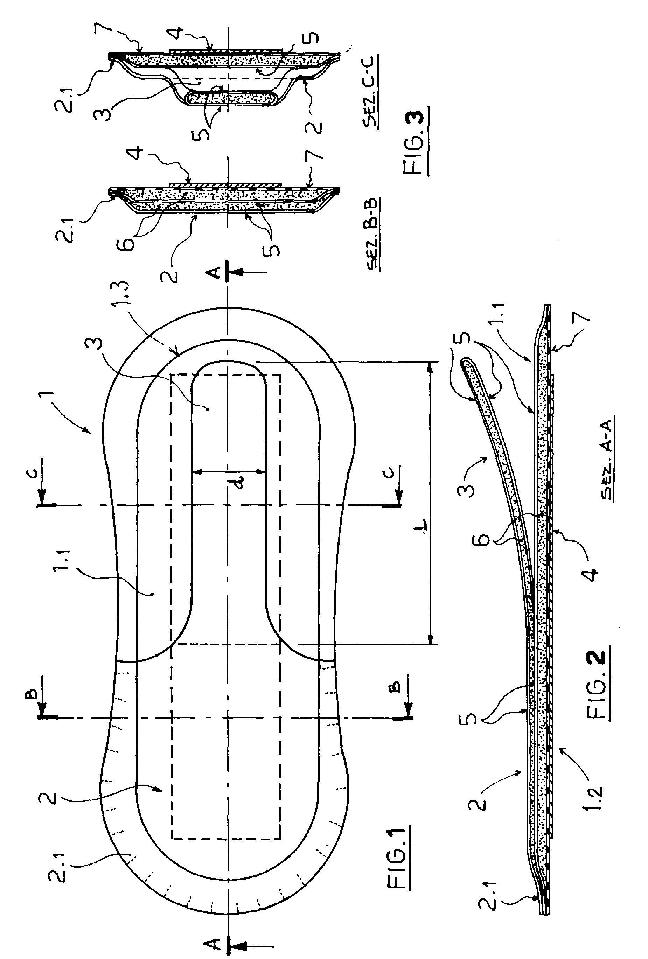

As we can note in fig. 1, the sanitary towel is made of two basic parts; an inferior layer made of a common sanitary towel 1 and a thinner superior layer 2 made of a super absorbent material.

From the superior layer 2, that has an area about nearly half of the inferior layer 1, a small strip extends having a width d of some centimetres and length L permitting it to reach the posterior border of the inferior layer 1.

The superior layer 2 is heat sealed to the inferior layer 1 along the borders 2.1; the lower surface 1.2 of the inferior layer 1 is covered, like in common sanitary towels, with a plastic waterproof film 7 with an adhesive 4.

The lower surface of the layer 2 and the strip 3 to it incorporated, is not covered by the waterproof and adhesive film; the absorbent layer of the strip 3 is instead totally covered i.e. incorporated in all its length with the filtering fabric 5 - see cross-section C-C.

As seen in fig. 2, the inferior surface of the strip 3, not being provided with a plastic adhesive film, does not stick to the surface 1.1 so that it can be raised up.

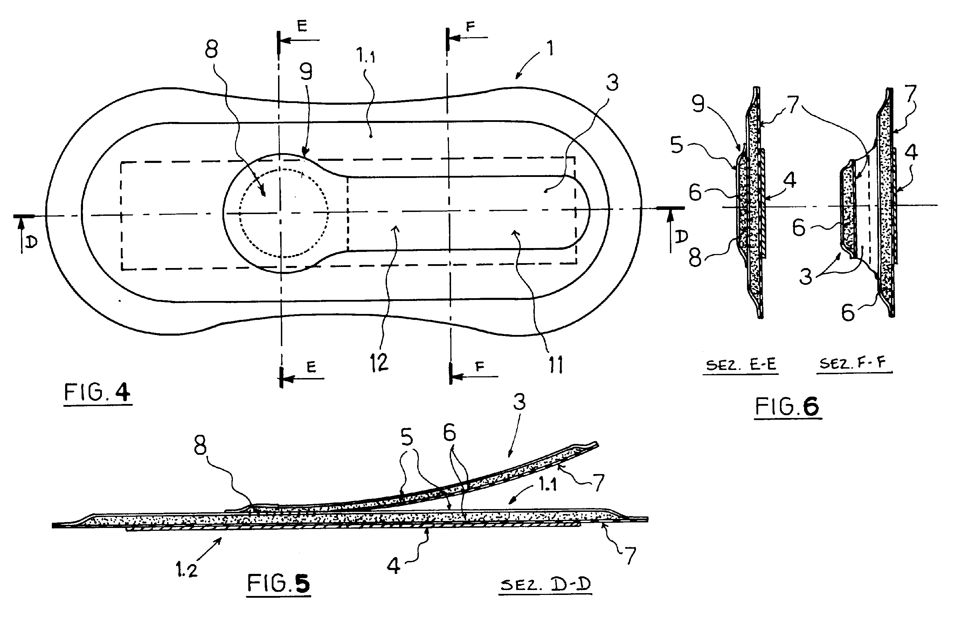

In the embodiment of fig. 4, the thin layer 2 that covers the superior surface of the layer 1 is missing and the anterior extremity 9 of the strip 3 is sticked to the superior surface 1.1 of the layer 1 by means of a suitable adhesive or heat seal 8, so that the strip can be raised up.

In this embodiment, the inferior surface of the strip 3 is covered by a waterproof film 7 whose's inferior surface is not adhesive, and the superior surface of said strip 3 is covered with a filtering fabric 5 that, along the borders, is sealed with the plastic film 7.

The cross sections E-E and F-F of fig. 4 clarify the composition of strip 3.

In the cross section E-E of fig. 6, we note in fact the absorbent layer 6 with filtering layer 5 on the superior surface, and the waterproof film 7 on the inferior surface are sealed together along the borders.

In the longitudinal cross section D-D of fig. 5, the strip 3 is raised up from the surface 1.1 being free of adhesive.

In this embodiment, the plastic film 7 forms a canalisation of the flow towards the anterior part of the sanitary towel.

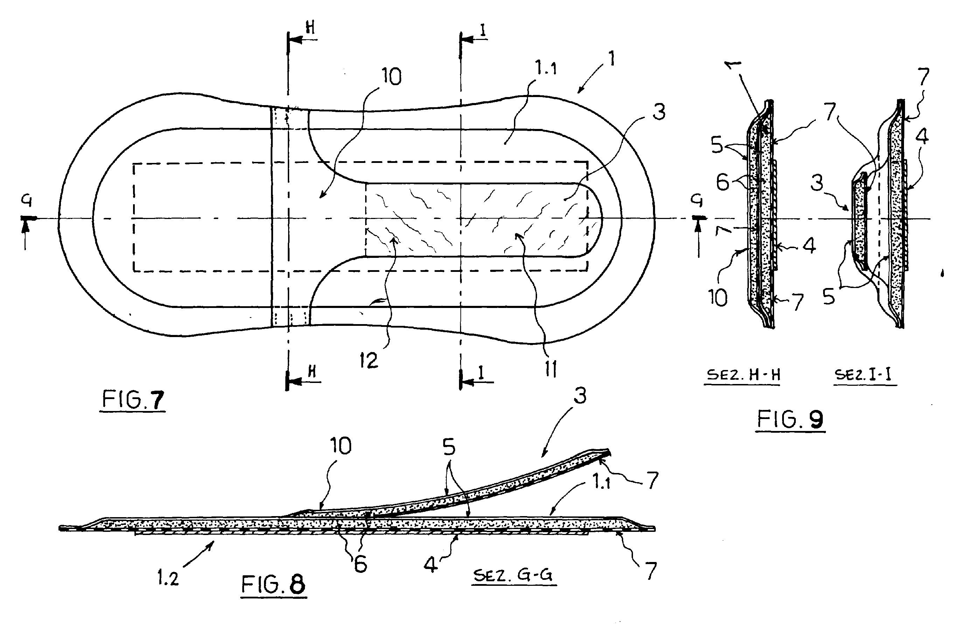

In fig. 7, the method of application of strip 3 to the lower layer 1 is different.

The anterior extremity of strip 3 is attached to the superior surface 1.1 of layer 1 by means of an extension 10 whose axis H-H is normal to the longitudinal axis G-G of strip 3, forming a figure like a T.

The strip 3 of the embodiment of fig. 7 is covered on its inferior surface with a waterproof film 7 as strip 3 in the embodiment of fig. 4.

In this case also, the inferior surface of the strip isn't provided with adhesive and can therefore adapt itself freely to the body, without being connected with the surface of layer 1.1.

In a further embodiment of the sanitary towels illustrated in figures 4 and 7, the inferior surface of the extremity part 11 of strip 3 is not provided with any waterproof film to facilitate the absorption of the flow from the surface 1. I of layer 1, reducing the flow directed towards the posterior borders.

The anterior part 12 of same strip 3, is instead provided with a waterproof film that must be positioned in correspondence of the outlet channel of the flow, in order to carry out better its function of containment.

The strip 3, due to its form and position, reduces the flow of the menstrual blood when the woman is lying on her back, permitting the inferior layer 1 of the sanitary towel to carry out its function without being overloaded.

## Claims

1. "Sanitary towel particularly suitable in preventing the menstrual flow to penetrate between the anal area", the sanitary towel, being particularly functional when the woman lies on her back, characterised by an inferior absorbent layer (1) to which is superimposed, without being sticked, on the part to be positioned posteriorly an absorbent strip (3) with width d of some centimetres and a length L permitting it to reach the proximity of the posterior border (1.3) of the layer (1), said strip (3) being attached to the inferior layer (1) with suitable means only in proximity of its intermediate area, and being covered or incorporated in the filtering fabric (5), and the inferior surface (1.2) of the layer (1) being covered by a waterproof film (7) with adhesive (4) applied on its inferior external surface.

2. "Sanitary towel particularly suitable in preventing the menstrual flow to penetrate between the anal area", as claimed in claim 1, characterised by a layer (1) on which is superimposed, on the part to be positioned anteriorly, a thinner layer of super absorbent material (2) sealed along the perimetrical border (2.1) to the perimetrical border of said inferior layer (1), the area of layer (2) being nearly half of the area of the inferior layer (1), and the absorbent strip (3) being an extension of said superimposed layer (2), strip (3) being not sticked to the surface (1.1) of layer (1)

3. Sanitary towel as claimed in claim 1, characterised by the fact that the anterior extremity (9) of the strip (3) attached with adhesive or other suitable sealing means on the superior surface (1.1) of the layer (1) has a circular shape.

4. Sanitary towel as claimed in claim 1, characterised by the fact that the anterior part (10) of the strip (3), attached with an adhesive or other suitable sealing means to the superior surface (1.1) of the layer (1), has the form of a rectangular strip forming with the strip a figure T.

5. Sanitary towel as claimed in claims 1, 2, 3 and 4, characterised by a waterproof film (7) which covers the inferior surface of strip (3).

6. Sanitary towel as claimed in claims 1, 2, 3 and 4, characterised by a strip (3) without a waterproof film on its inferior surface.

7. Sanitary towel as claimed in claims 1, 3 and 4, characterised by a waterproof film (7) which covers only the anterior part (12) of the inferior surface of the strip (3) and by the fact the other half (11) of the inferior surface of the same strip is free from said waterproof film.
